# EUROPÄISCHE PATENTSCHRIFT

(11) **EP 1 288 660 B1**
(45) Veröffentlichungstag und Bekanntmachung des Hinweises auf die Patenterteilung: **21.11.2007**
(21) Anmeldenummer: 02018318.2
(22) Anmeldetag: 14.08.2002
(51) Int. Cl.: G01N 33/49, G01N 11/02

(54) **Anordnung und Verfahren zur Untersuchung der Fliessfähigkeit einer physiologischen Flüssigprobe**
Method and apparatus for investigating the flow properties of a physiological sample
Méthode et appareil pour étudier les propriétés d'écoulement d'un échantillon physiologique

(30) Priorität: 24.08.2001 DE 10140699
(43) Veröffentlichungstag der Anmeldung: 05.03.2003
(73) Patentinhaber: Roche Diagnostics GmbH, 68305 Mannheim (DE); F. HOFFMANN-LA ROCHE AG, 4070 Basel (CH)
(72) Erfinder: Harttig, Herbert, Dr., 67122 Altrip (DE)
(74) Vertreter: Pfiz, Thomas

(56) Entgegenhaltungen:
- DE-A1- 1 598 514
- DE-A1- 3 218 515
- SU-A1- 749 386
- US-A- 5 110 727
- POPOV E G: "ORIENTATION OF NONSPHERICAL CELLS IN BLOOD FLOWING THROUGH A VESSEL" BULLETIN OF EXPERIMENTAL BIOLOGY AND MEDICINE, Bd. 86, Nr. 11, 1978, Seiten 1556-1557, XP008062411 ISSN: 0007-4888

## Beschreibung

Die Erfindung betrifft eine Anordnung und ein Verfahren zur Untersuchung der Fließfähigkeit einer physiologischen Flüssigprobe, insbesondere einer Blutprobe für Koagulationstests.

Aus der US-A-5 110 727 ist ein Koagulationstestsystem dieser Art bekannt. Damit sollen mit einfachen Mitteln Blutgerinnungs- oder Gerinnungsauflösungstests *ex vivo* ermöglicht werden, beispielsweise um die Wirkung von Anti-Koagulanzien zu überwachen. Als Messpartikel werden dort Eisenoxidteilchen eingesetzt, die in einer Messkapillare in der Probe dispergiert und einem oszillierenden Magnetfeld unterworfen werden. Dies führt dazu, dass die Messpartikel im Takt der Magnetfeldänderungen sich bewegen und entsprechend die Transparenz bzw. Reflektivität im Messbereich einer Detektoreinheit sich ändert. Mit zunehmender Probengerinnung wird die Teilchenbewegung unterbunden, und das Messsignal bleibt konstant. Als Aktuator dient dabei ein Magnetsystem bestehend aus einem Vorspann-Permanentmagnet und einem mit Wechselspannung betriebenen Elektromagneten. Damit die notwendigen Flussdichteänderungen erreicht werden, muss mit relativ großen Spulen und hohen Strömen gearbeitet werden, was sich vor allem bei einem tragbaren Gerät hinsichtlich des Gewichts und der Stromversorgung als nachteilig erweist.

Die DE 32 18 515 A1 offenbart eine Methode zur Messung der Blutgerinnungszeit bei dem ein Probe-Reagenz-Gemisch in einer stillstehenden Küvette so bewegt wird, dass an einer in die Küvette hineinragenden Kante ein optisch erfasstes Blutgerinnsel gebildet wird, wobei ein den Inhalt der Küvette bewegender variabler Luftdruck erzeugt wird.

Ausgehend hiervon liegt der Erfindung die Aufgabe zugrunde, die Nachteile des Standes der Technik zu vermeiden und ein Meßsystem der eingangs angegebenen Art dahingehend zu verbessern, dass bei einfacher Handhabung mit geringem Geräteaufwand zuverlässige und reproduzierbare Messergebnisse erzielt werden.

Zur Lösung dieser Aufgabe wird die im Patentanspruch 1 bzw. 19 angegebene Merkmalskombination vorgeschlagen. Vorteilhafte Ausgestaltungen und Weiterbildungen der Erfindung ergeben sich aus den abhängigen Ansprüchen.

Im Hinblick auf ein Meßsystem wird erfindungsgemäß vorgeschlagen, dass die Aktuatoreinrichtung durch eine Pumpeinheit zur Erzeugung einer den Orientierungswechsel der Messpartikel vermittelnden, längs des Aufnahmekanals hin- und hergehenden Strömung der Flüssigprobe gebildet ist. Damit ist es möglich, auch nichtmagnetische Messpartikel einzusetzen, welche im Hinblick auf das Detektionsverfahren weiter optimiert sein können. Eine oszillierende Strömung lässt sich im Gegensatz zu einem magnetischen Wechselfeld mit geringem Energieverbrauch und einfachen baulichen Mitteln erzeugen. Das erfindungsgemäße System erfordert keine aufwendige Manipulation und ermöglicht mit kleinsten Probenmengen einen automatischen Messablauf. Da die Messpartikelbewegung durch eine Wechselströmung induziert wird, spielen Fertigungstoleranzen und die inneren Oberflächeneigenschaften des Probenaufnahmekanals eine untergeordnete Rolle, und die Anfälligkeit gegen etwaige Luftblaseneinschlüsse wird minimiert/Zur automatischen Bestimmung eines Testparameters nach Maßgabe des zeitabhängig erfassten Orientierungswechsels der Messpartikel ist eine Auswerteeinheit vorgesehen.

Zur zyklisch wechselnden Saug- und Druckbeaufschlagung der Flüssigprobe in dem Aufnahmekanal sieht eine bevorzugte Ausführung vor, dass die Pumpeinheit ein insbesondere mechanisch, pneumatisch oder piezoelektrisch betätigtes Verdrängungsorgan aufweist. Eine weitere bauliche Vereinfachung lässt sich dadurch erzielen, dass die Pumpeinheit eine den Aufnahmekanal partiell begrenzende flexible Verdrängermembran aufweist. Zur effektiven Strömungserzeugung sollte die Pumpeinheit an einer Pumpstelle im Bereich eines geschlossenen oder verschließbaren Endabschnitts des Aufnahmekanals angeschlossen sein.

In fertigungstechnischer Hinsicht insbesondere für größere Stückzahlen ist es vorteilhaft, wenn der Aufnahmekanal durch eine Ausnehmung in einer Zwischenlage eines mehrlagigen Folienverbundkörpers gebildet ist.

Eine weitere vorteilhafte Ausführung der Erfindung sieht vor, dass der Aufnahmekanal zumindest abschnittsweise als Kapillarkanal ausgebildet ist. Damit lassen sich geringste Probenmengen untersuchen, und die Probenflüssigkeit kann selbstansaugend aufgenommen werden. Zudem werden über einen Kapillarquerschnitt hohe Strömungsgradienten und somit eine effektive Ausrichtung der Messpartikel erreicht.

Eine weitere Verbesserung wird dadurch erreicht, dass die Messpartikel eine von der Kugelform abweichende, langgestreckte und/oder abgeflachte Gestalt aufweisen. Dadurch richten sich die Teilchen im Scherfeld der Probenströmung stapelartig quer zur Strömungsrichtung aus, so dass letztlich eine hohe Nachweisempfindlichkeit erreicht werden kann. Hierbei ist es günstig, wenn das Verhältnis von Längs- zu Querabmessung der Messpartikel größer als 2, vorzugsweise größer als 10 ist. Die Messpartikel sind bevorzugt als Pigmente, vorzugsweise als plättchenförmige Effektpigmente ausgebildet, oder als stäbchenförmige oder faserförmige Gebilde, insbesondere als Whiskers.

Um störende Einflüsse auf die Probe zu reduzieren, ist es vorteilhaft, wenn die Messpartikel durch eine polymere oder oligomere Schutzschicht ummantelt sind.

In Flüssigkeitsproben ohne feste Teilchen werden die Messpartikel als Fremdsubstanz suspendiert, während es andernfalls auch denkbar ist, dass die Messpartikel durch Probenbestandteile, insbesondere Erythrozyten in einer Vollblutprobe, gebildet sind.

In bevorzugter Ausführung umfasst die Detektoreinrichtung eine Strahlungsquelle und einen Strahlungsempfänger für elektromagnetische Strahlung, vorzugsweise eine Leuchtdiode und einen optoelektronischen Halbleitersensor. Der Nachweis beruht vorteilhafterweise darauf, dass die Messpartikel eingestrahlte elektromagnetische Strahlung in Abhängigkeit vom Einstrahlwinkel unterschiedlich absorbieren und/oder reflektieren. Zur direkten Detektion sollte die Wandung des Aufnahmekanals zumindest bereichsweise für die Messstrahlung der Detektoreinrichtung durchlässig sein.

Um reproduzierbare Messbedingungen zu schaffen, ist es von Vorteil, wenn der Aufnahmekanal mit einer Temperiereinrichtung zur Einstellung einer vorgegebenen Temperatur der Flüssigprobe gekoppelt ist.

Zur Durchführung von Koagulationstests wird speziell eine Messanordnung mit einem Kapillarkanal zur Aufnahme einer Messpartikel enthaltenden oder damit versetzten Blutprobe, einer Pumpeinheit zur Erzeugung einer die Orientierung der Messpartikel zyklisch variierenden, längs des Aufnahmekanals hin- und hergehenden Strömung der Blutprobe und einer Detektoreinrichtung zur vorzugsweise optischen Erfassung des Orientierungswechsels der Messpartikel vorgeschlagen.

In verfahrensmäßiger Hinsicht wird die vorstehend genannte Aufgabe dadurch gelöst, dass eine in dem Aufnahmekanal hin- und hergehende Strömung der Flüssigprobe zur zyklisch veränderlichen Ausrichtung der Messpartikel erzeugt wird. Die Flüssigprobe wird in dem Aufnahmekanal einer Scherströmung unterworfen, so dass die Messpartikel je nach Strömungsrichtung im Gleichtakt unterschiedlich ausgerichtet werden.

In bevorzugter Ausgestaltung wird der Orientierungswechsel der Messpartikel als alternierende Änderung im Reflexions- oder Absorptionsvermögen der Flüssigprobe mittels einer optischen Detektoreinrichtung erfasst.

Zur Durchführung von Koagulationstests wird der Orientierungswechsel der Messpartikel über eine Vielzahl von Zyklen aufgezeichnet und zur Bestimmung einer Kenngröße, insbesondere der Prothrombinzeit (PT), der aktivierten Gerinnungszeit (ACT) oder der aktivierten, partiellen Thromboplastinzeit (aPTT) zeitabhängig ausgewertet.

Im Folgenden wird die Erfindung anhand eines in der Zeichnung in schematischer Weise dargestellten Ausführungsbeispiels näher erläutert. Es zeigen
- Fig. 1: ein Blockschaltbild einer Anordnung bzw. eines Meßsystems zur Durchführung von Koagulationstests an Blutproben;
- Fig. 2: das Meßsystem in schaubildlicher Darstellung;
- Fig. 3 und 4: einen Probenträger des Meßsystems mit einem Aufnahmekanal für eine Blutprobe in der Draufsicht und im Vertikalschnitt;
- Fig. 5: eine ausschnittsweise Darstellung des Aufnahmekanals mit darin befindlichen Messpartikeln bei hin- und hergehender Strömung;
- Fig. 6: ein Zeitdiagramm eines den Orientierungswechsel der Messpartikel widerspiegelnden Messsignals; und
- Fig. 7: einen zeitgedehnten Ausschnitt des Signalverlaufs nach Fig. 6.

Die in der Zeichnung dargestellte Messanordnung zur Durchführung von Koagulationstests an Blutproben besteht im wesentlichen aus einem Probenträger 10 mit einem Aufnahmekanal 12 für eine mit Messpartikeln 14 versatz te Blutprobe 16, einer Pumpeinheit 18 zur Erzeugung einer hin- und hergehenden Strömung der Blutprobe in dem Aufnahmekanal und einer Detektoreinrichtung 20, 22 zur optischen Erfassung des strömungsinduzierten Orientierungswechsels der Messpartikel 14 als mittelbares Viskositätsmaß.

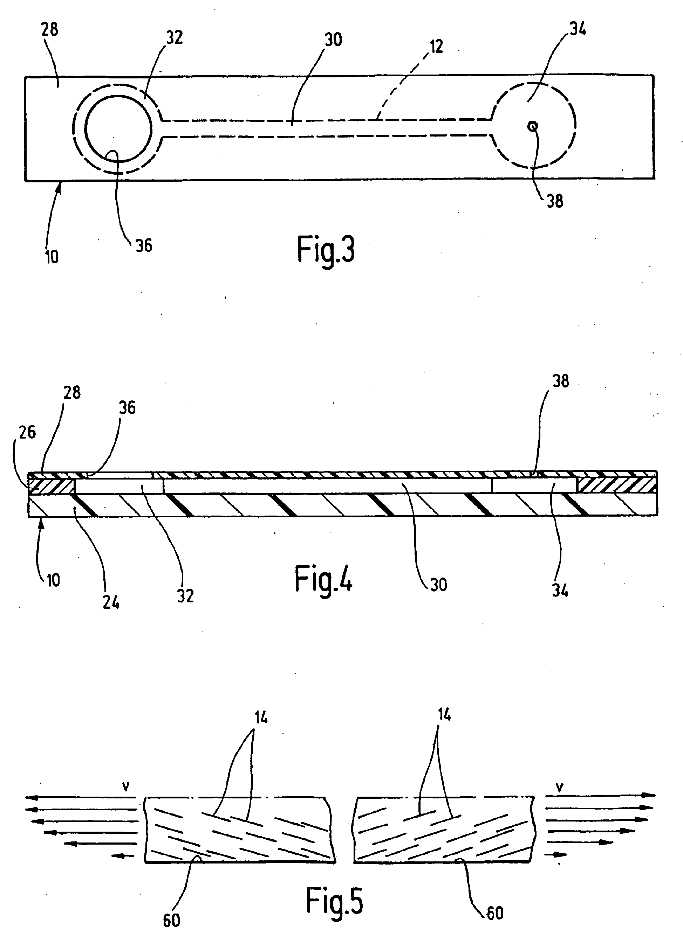

Der in Fig. 3 und 4 gezeigte Probenträger 10 weist als mehrlagiger Kunststoffverbundkörper eine biegesteife Bodenfolie 24, eine Zwischenfolie 26 und eine flexible, transparente Deckfolie 28 auf. In der Zwischenfolie 26 ist eine den Aufnahmekanal 12 bildende Ausnehmung ausgestanzt, welche einen kapillarförmigen Kanalabschnitt 30 und mündungsseitig daran anschließend eine Probenaufgabekammer 32 und eine Pumpkammer 34 aufweist. Die Probenaufgabekammer 32 lässt sich über eine Einlassöffnung 36 in der Deckfolie 28 beladen, während die Pumpkammer 34 über eine Entlüftungsöffnung 38 in der Deckfolie 28 mit Atmosphäre kommuniziert.

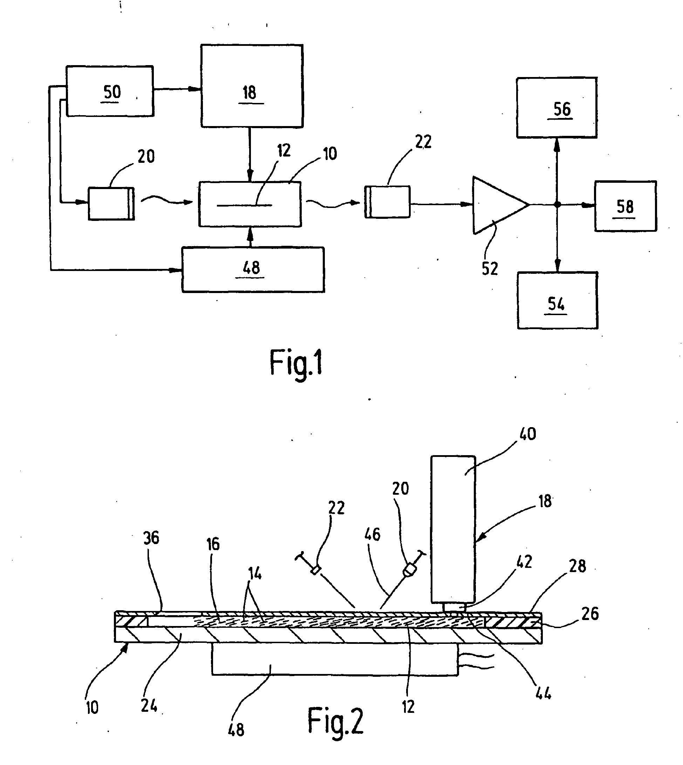

Wie in Fig. 2 veranschaulicht, kann die Pumpeinheit 18 durch einen mittels Hubantrieb 40 angetriebenen Stempel 42 aufweisen, welcher über der Pumpkammer 34 unter Verschluss der Entlüftungsöffnung 38 auf die Deckfolie 28 aufsetzbar ist. Die flexible Deckfolie 28 wirkt dabei als Verdrängungsorgan 44 zur zyklisch wechselnden Saug- und Druckbeaufschlagung der Blutprobe in dem Aufnahmekanal 12. Es versteht sich, dass auch andere, insbesondere pneumatisch oder piezoelektrisch arbeitende Betätigungsmittel zur Erzeugung einer hin- und hergehenden Flüssigkeitsströmung einsetzbar sind.

Die Detektoreinrichtung umfasst eine Leuchtdiode 20 als Strahlungsquelle, welche durch die Deckfolie 28 hindurch in den Kapillarabschnitt 30 des Aufnahmekanals 12 einstrahlt, sowie einen entsprechend ausgerichteten Photosensor 22 zur Erfassung der durch die Blutprobe reflektierten oder durch diese hindurchgestrahlten Messstrahlung 46.

Wie nachstehend näher erläutert, beruht das Messprinzip darauf, dass durch die Druckwechselbelastung der Blutprobe die Orientierung der darin befindlichen Messpartikel in Abhängigkeit von der Probenviskosität wechselt, was sich über die Änderung der optischen Eigenschaften im Strahlengang der Detektoreinrichtung 20, 22 nachweisen lässt. Solange die Blutprobe in Bewegung ist, fluktuieren die optischen Eigenschaften. Tritt Koagulation ein, so bleiben sie schließlich konstant.

Um die Flüssigprobe auf eine definierte Messtemperatur zu bringen, ist eine Heizeinrichtung 48 vorgesehen, welche über die Bodenfolie 24 mit dem Aufnahmekanal 12 in Wärmeleitkontakt steht.

Die Steuerung des Messablaufs erfolgt über eine ausgangsseitig mit den Einheiten 18, 20, 48 verbundene Steuereinrichtung 50.

Zur weiteren Verarbeitung und Auswertung der Messsignale des Photosensor 22 sind in einer Auswerteschaltung ein Messverstärker 52, ein Signalrecorder 54, eine Auswerteeinheit 56 mit Echtzeituhr und eine Anzeigeeinheit 58 vorgesehen. Die Auswerteeinheit 56 umfasst einen dem Messverstärker 52 nachgeordneten A/D-Wandler zur zeitabhängigen Digitalisierung des Messsignals und eine als Microcontroller ausgebildete digitale Verarbeitungseinheit zur Bestimmung von Testparametern aus dem Signalverlauf. Die gesamte Messanordnung lässt sich in einem tragbaren batteriebetriebenen Handgerät unterbringen, um die vorgesehenen Tests ortsunabhängig durchführen zu können.

Wie aus Fig. 5 ersichtlich, sind die Messpartikel 14 in dem Kapillarabschnitt 30 des Aufnahmekanals 12 einer Scherströmung unterworfen, wie sie für die beiden gezeigten Stromrichtungen durch die Strömungspfeile veranschaulicht ist. Die Strömungsgeschwindigkeit v ist also in der Kanalmitte am großten, zur Kanalwand 60 hin nimmt sie bei laminarer Strömung in Form einer Parabel ab. Dies führt bei gegebener anisotroper Teilchengeometrie zu einer untereinander gleichen Querausrichtung der Messpartikel 14 mit ihrer langen Achse quer bzw. schräg zur momentanen Strömungsrichtung, wobei aufgrund der periodischen Strömungsumkehr ein entsprechender Orientierungswechsel stattfindet.

Als geeignete anisotrope Messpartikel sind bevorzugt plättchenförmige Pigmente und speziell Effektpigmente vorgesehen. Für die Detektion von Bedeutung ist die Eigenschaft dieser Pigmente, die eingestrahlte elektromagnetische Strahlung winkelabhängig zu reflektieren bzw. zu absorbieren, so dass ausgeprägte Helligkeitswechsel in Abhängigkeit vom jeweiligen Orientierungswinkel erhalten werden.

Interferenzeffektpigmente bestehen aus einem flachen Glimmerplättchen als Träger, das mit einem schwerbrechenden Metalloxid wie beispielsweise Titandioxid beschichtet ist. Bei geringer Schichtdicke ergeben sich silberweiße Farben, während mit steigender Schichtdicke gelbe, rote, blaue und schließlich grüne Perlglanzpigmente erhalten werden. Solche Pigmente werden von verschiedenen Herstellern geliefert, beispielsweise von der Firma Merck KGaA, Darmstadt, Deutschland unter dem Handelsnamen Iriodin.

Um Rückwirkungen auf das Koagulationsverhalten auszuschließen, kann die Oberfläche der eingesetzten Pigmente durch polymere oder oligomere Schutzschichten modifiziert sein. Dazu eignen sich ungeladene synthetische Polymere (wie Polyethylen, Polypropylen, Polysilicon, Polytetrafluorethylen, Polystyrol), hydrophile, ungeladene Polymere (wie Polyethylenglycol, Polyvinylpyrrolidon) oder positiv geladene Polymere, wie Heparinderivate. Diese Polymere oder Oligomere davon können auf verschiedene Weise auf der Pigmentoberfläche fixiert werden, insbesondere durch chemische Bindung oder durch einfaches Coating wie bei der Beschichtung mit Polystyrol aus Lösung. Besonders bevorzugt sind Schichten auf der Basis von Polyethylenglycol oder Polyvinylpyrrolidon.

Zur Durchführung eines Gerinnungstests ist der Aufnahmekanal 12 an seiner inneren Oberfläche mit einer Reagenzmischung beschichtet, worin neben den Messpartikeln 14 auch Thromboplastine enthalten sind. Ein ausreichendes Aliquot einer Blutprobe, beispielsweise 50 µl wird mittels eines Transferinstruments auf die Probenaufgabekammer 32 aufgebracht und selbsttätig durch Kapillarwirkung in den Aufnahmekanal 12 eingesaugt. Etwa vorhandene Luft entweicht dabei über die Entlüftungsöffnung 38. In dem einfließenden Blut wird die Reagenzmischung aufgenommen und dabei die Messpartikel (z.B. 0,1 mg Effektpigmente) verteilt. Um einen definierten Ablauf der komplexen enzymatischen Reaktionen sicherzustellen, wird mittels der Heizeinrichtung 48 eine konstante Temperatur beispielsweise von 39°C eingeregelt.

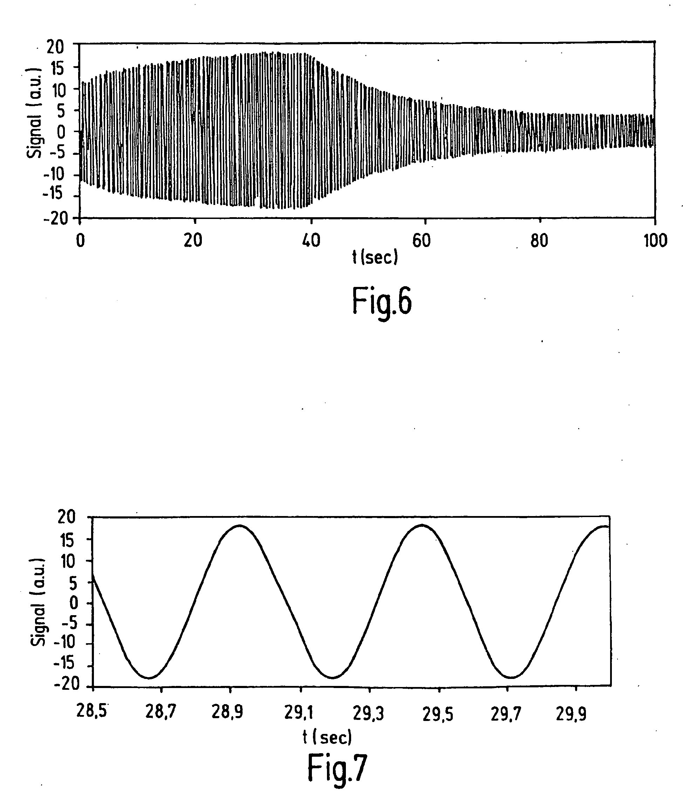

Zur Erzeugung der Wechselströmung wird mittels der Pumpeinheit 18 ein niederfrequenter Druck- und Saughubwechsel im Bereich von einigen Herz auf die Blutprobe in dem Kanal 12 ausgeübt. Durch die strömungsinduzierte Bewegung der Messpartikel 14 ändert sich die Helligkeit im Erfassungsbereich der Detektoreinrichtung 20, 22 mit der gleichen Frequenz. Entsprechend steht am Ausgang des Verstärkers 52 ein oszillierendes AC-Signal zur Verfügung, wie es in Fig. 6 und 7 in willkürlichen Einheiten über der Zeit dargestellt ist. Während eines anfänglichen Anstiegsintervalls werden die Messpartikel 14 im Scherfeld der Strömung zunehmend im Gleichtakt stapelförmig hin- und her bewegt. Sobald die Koagulation einsetzt, wird die Bewegung behindert und die Signalamplitude fällt entsprechend ab.

Aus dem Signalabfall lässt sich über einen geeigneten Algorithmus in der Auswerteeinheit 56 unter Berücksichtigung spezifischer Einflussgrößen und Skalierungsfaktoren ein Testparameter für die Blutgerinnungsreaktion wie die Prothrombinzeit bestimmen.

Es versteht sich, dass nach dem vorstehend beschriebenen Prinzip das Fließverhalten bzw. Viskositätsänderungen nicht nur an Blutproben, sondern in beliebigen Flüssigkeitsproben und insbesondere auch in anderen pysiologischen Fluiden wie Plasma, Speichel, Gewebeflüssigkeit, Milch etc. untersucht werden kann. Zur Modifikation von Eigenschaften des Mediums können Additive wie Benetzungs- und Konservierungsmittel, Aktivatoren und Puffer zugesetzt werden. Grundsätzlich ist es möglich, dass die Probe selbst die messbare Komponente liefert, beispielsweise indem Erythrozyten als scheibchenförmige Messpartikel in einer Blutprobe genutzt werden. Denkbar ist es auch, anstelle einer strahlenoptischen Erfassung der Partikelbewegung andere berührungslose, beispielsweise kapazitiv arbeitende Detektionsverfahren einzusetzen.

## Patentansprüche

1. Anordnung zur Untersuchung der Fließfähigkeit einer physiologischen Flüssigprobe, insbesondere einer Blutprobe für Koagulationstests, mit einem Aufnahmekanal (12) für die Flüssigprobe (16), einer Aktuatoreinrichtung (18) zur zyklischen Änderung der Orientierung von Messpartikeln (14) in der Flüssigprobe (16), wobei die Aktuatoreinrichtung durch eine Pumpeinheit (18) zur Erzeugung einer den Orientierungswechsel der Messpartikel (14) vermittelnden, längs des Aufnahmekanals (12) hin- und hergehenden Strömung der Flüssigprobe (16) gebildet ist und einer Detektoreinrichtung (20, 22) zur vorzugsweise optischen Erfassung des Orientierungswechsels der Messpartikel (14) **gekennzeichnet durch** eine Auswerteeinheit (56) eingerichtet für die Bestimmung eines Testparameters nach Maßgabe des zeitabhängig erfassten Orientierungswechsels der Messpartikel (14).

2. Anordnung nach Anspruch 1, **dadurch gekennzeichnet, dass** die Pumpeinheit (18) ein insbesondere mechanisch, pneumatisch oder piezoelektrisch betätigtes Verdrängungsorgan (44) zur zyklisch wechselnden Saug- und Druckbeaufschlagung der Flüssigprobe (16) in dem Aufnahmekanal (12) aufweist.

3. Anordnung nach Anspruch 1 oder 2, **dadurch gekennzeichnet, dass** die Pumpeinheit (18) eine den Aufnahmekanal (12) partiell begrenzende flexible Verdrängermembran (28) aufweist.

4. Anordnung nach einem der Ansprüche 1 bis 3, **dadurch gekennzeichnet, dass** die Pumpeinheit (18) an einer Pumpstelle im Bereich eines geschlossenen oder verschließbaren Endabschnitts (34) des Aufnahmekanals (12) angeschlossen ist.

5. Anordnung nach einem der Ansprüche 1 bis 4, **dadurch gekennzeichnet, dass** der Aufnahmekanal (12) durch eine Ausnehmung in einer Zwischenlage (26) eines mehrlagigen Folienverbundkörpers (10) gebildet ist.

6. Anordnung nach einem der Ansprüche 1 bis 5, **dadurch gekennzeichnet, dass** der Aufnahmekanal (12) zumindest abschnittsweise als Kapillarkanal (30) ausgebildet ist.

7. Anordnung nach einem der Ansprüche 1 bis 6, **dadurch gekennzeichnet, dass** die Messpartikel (14) eine von der Kugelform abweichende, langgestreckte und/oder abgeflachte Gestalt aufweisen.

8. Anordnung nach einem der Ansprüche 1 bis 7, **dadurch gekennzeichnet, dass** das Verhältnis von Längs- zu Querabmessung der Messpartikel (14) größer als 2, vorzugsweise größer als 10 ist.

9. Anordnung nach einem der Ansprüche 1 bis 8, **dadurch gekennzeichnet, dass** die Messpartikel (14) als plättchenförmige Pigmente, vorzugsweise als Effektpigmente ausgebildet sind.

10. Anordnung nach einem der Ansprüche 1 bis 8, **dadurch gekennzeichnet, dass** die Messpartikel (14) als stäbchenförmige oder faserförmige Gebilde insbesondere als Whiskers ausgebildet sind.

11. Anordnung nach einem der Ansprüche 1 bis 10, **dadurch gekennzeichnet, dass** die Messpartikel (14) durch eine polymere oder oligomere Schutzschicht ummantelt sind.

12. Anordnung nach einem der Ansprüche 1 bis 11, **dadurch gekennzeichnet, dass** die Messpartikel (14) als Fremdsubstanz in der Flüssigprobe (16) suspendiert sind.

13. Anordnung nach einem der Ansprüche 1 bis 11, **dadurch gekennzeichnet, dass** die Messpartikel (14) durch Probenbestandteile der Flüssigprobe (16), insbesondere Erythrozyten in einer Vollblutprobe, gebildet sind.

14. Anordnung nach einem der Ansprüche 1 bis 13, **dadurch gekennzeichnet, dass** die Detektoreinrichtung (20, 22) eine Strahlungsquelle (20) und einen Strahlungsempfänger (22) für elektromagnetische Strahlung umfasst.

15. Anordnung nach einem der Ansprüche 1 bis 14, **dadurch gekennzeichnet, dass** die Detektoreinrichtung (20, 22) eine Leuchtdiode (20) und einen optoelektronischen Halbleitersensor (22) umfasst.

16. Anordnung nach einem der Ansprüche 1 bis 15, **dadurch gekennzeichnet, dass** die Messpartikel (14) eingestrahlte elektromagnetische Strahlung in Abhängigkeit vom Einstrahlwinkel unterschiedlich absorbieren und/oder reflektieren.

17. Anordnung nach einem der Ansprüche 1 bis 16, **dadurch gekennzeichnet, dass** die Wandung des Aufnahmekanals (12) zumindest bereichsweise für die Messstrahlung der Detektoreinrichtung (20, 22) durchlässig ist.

18. Anordnung nach einem der Ansprüche 1 bis 17, **dadurch gekennzeichnet, dass** der Aufnahmekanal (12) mit einer Temperiereinrichtung (48) zur Einstellung einer vorgegebenen Temperatur der Flüssigprobe (16) gekoppelt ist.

19. Verfahren zur Untersuchung der Fließfähigkeit einer physiologischen Flüssigprobe, insbesondere einer Blutprobe für Koagulationstests, bei welchem eine Flüssigprobe (16) in einen Aufnahmekanal (12) eingebracht wird, in der Flüssigprobe (16) verteilte Messpartikel (14) zyklisch unterschiedlich ausgerichtet werden, und der Orientierungswechsel der Messpartikel (14) vorzugsweise optisch erfasst wird, **dadurch gekennzeichnet, dass** eine in dem Aufnahmekanal (12) hin- und hergehende Strömung der Flüssigprobe (16) zur zyklisch veränderlichen Ausrichtung der Messpartikel (14) erzeugt wird.

20. Verfahren nach Anspruch 19, **dadurch gekennzeichnet, dass** die Flüssigprobe (16) in dem Aufnahmekanal (12) einer Scherströmung unterworfen wird, so dass die Messpartikel (14) je nach Strömungsrichtung unterschiedlich ausgerichtet werden.

21. Verfahren nach Anspruch 19 oder 20, **dadurch gekennzeichnet, dass** der Orientierungswechsel der Messpartikel (14) als alternierende Änderung im Reflexions- oder Absorptionsvermögen der Flüssigprobe (16) mittels einer optischen Detektoreinrichtung (20, 22) erfasst wird.

22. **Verfahren nach einem der Ansprüche 19 bis 21, dadurch gekennzeiehnet, dass der Orientierungswechsel der Messpartikel (14) über** eine Vielzahl von Zyklen aufgezeichnet und zur Bestimmung einer Kenngröße, insbesondere der Prothrombinzeit (PT), der aktivierten Gerinnungszeit (ACT) oder der aktivierten, partiellen Thromboplastinzeit (aPTT) zeitabhängig ausgewertet wird.

## Claims

1. Device for investigating the flowability of a physiological fluid sample, in particular a blood sample for coagulation tests, having an uptake passage (12) for the fluid sample (16), an actuator device (18) for the cyclic change in orientation of measuring particles (14) in the fluid sample (16), the actuator device being formed by a pump unit (18) for producing a flow of the fluid sample (16) that travels back and forth along the capillary passage (12) and provides the cyclic change in orientation of the measuring particles (14), and a detector device (20, 22) to preferable optically detect the change in orientation of the measuring particles (14), **characterized by** an evaluation unit (56) being set up for determining a test parameter according to the change in orientation of the measuring particles (14) detected as a function of time.

2. Device according to claim 1, **characterized in that** the pump unit (18) comprises an, in particular, mechanically, pneumatically or piezoelectrically-actuated displacer (44) for acting upon the fluid sample (16) in the uptake passage (12) with suction and pressure in a cyclically changing fashion.

3. Device according to claim 1 or 2, **characterized in that** the pump unit (18) comprises a flexible displacement membrane (28) that partially borders the uptake passage (12).

4. Device according to one of the claims 1 to 3, **characterized in that** the pump unit (18) is connected to a pump site in the region of a closed or closeable end portion (34) of the uptake passage (12).

5. Device according to one of the claims 1 to 4, **characterized in that** the uptake passage (12) is formed by a recess in an intermediate layer (26) of a multi-layer plastic sheet interconnecting body (10).

6. Device according to one of the claims 1 to 5, **characterized in that** the uptake passage (12) is designed as a capillary passage (12), at least in sections.

7. Device according to one of the claims 1 to 6, **characterized in that** the measuring particles (14) are non-spherical, long-straggling and/or flattened in shape.

8. Device according to one of the claims 1 to 7, **characterized in that** the ratio of the longitudinal dimension to the transverse dimension of the measuring particles (14) is greater than 2, preferably greater than 10.

9. Device according to one of the claims 1 to 8, **characterized in that** the measuring particles (14) are designed as platelet-shaped pigments, preferably as effect pigments.

10. Device according to one of the claims 1 to 8, **characterized in that** the measuring particles (14) are designed as rod-shaped or fiber-shaped formations, in particular as whiskers.

11. Device according to one of the claims 1 to 10, **characterized in that** the measuring particles (14) are covered with a polymeric or oligomeric protective layer.

12. Device according to one of the claims 1 to 11, **characterized in that** the measuring particles (14) are suspended in the fluid sample (16) as a foreign substance.

13. Device according to one of the claims 1 to 11, **characterized in that** the measuring particles (14) are formed by sample components of the fluid sample (16), in particular erythrocytes in a whole blood sample.

14. Device according to one of the claims 1 to 13, **characterized in that** the detector device (20, 22) includes a radiation source (20) and a radiation receiver (22) for electromagnetic radiation.

15. Device according to one of the claims 1 to 14, **characterized in that** the detector device (20, 22) includes a light diode (20) and an optoelectronic semiconductor sensor (22).

16. Device according to one of the claims 1 to 15, **characterized in that** the measuring particles (14) absorb and/or reflect differently incident electromagnetic radiation depending on the angle of incidence.

17. Device according to one of the claims 1 to 16, **characterized in that** the wall of the uptake passage (12) is permeable to the measuring radiation of the detector device (20, 22), at least in sections.

18. Device according to one of the claims 1 to 17, **characterized in that** the uptake passage (12) is coupled to a tempering device (48) for adjusting a specified temperature of the fluid sample (16).

19. Method for investigating the flowability of a physiological fluid sample, in particular a blood sample for coagulation tests, in which a fluid sample (16) is brought into a take-up passage (12), measuring particles (14) distributed in the fluid sample (16) are oriented differently in cyclic fashion, and the change in orientation of the measuring particles (14) is detected preferably optically, **characterized in that** a flow of the fluid sample (16) travelling back and forth in the uptake passage (12) is produced for the cyclically changeable orientation of the measuring particles (14).

20. Method according to claim 19, **characterized in that** the fluid sample (16) is subjected to a shear flow in the uptake passage (12), so that the measuring particles (14) are oriented differently depending on the direction of flow.

21. Method according to claim 19 or 20, **characterized in that** the change in orientation of the measuring particles (14) is detected as an alternating change in the reflectance or absorption capability of the fluid sample (16) by means of an optical detector device (20, 22).

22. The method according to one of the claims 19 to 21, **characterized in that** the change in orientation of the measuring particles (14) is recorded via a plurality of cycles, and is evaluated as a function of time to determine a variable, in particular the prothrombin time (PT), the activated clotting time (ACT) or the activated partial thromboplastin time (aPTT).

## Revendications

1. Appareil pour étudier les propriétés d'écoulement d'un échantillon liquide physiologique, plus particulièrement d'un échantillon de sang pour des tests de coagulation, avec un canal de réception (12) pour l'échantillon liquide (16), un dispositif d'actionnement (18) destiné à la modification cyclique de l'orientation de particules de mesure (14) dans l'échantillon liquide (16), moyennant quoi le dispositif d'actionnement est formé par une unité de pompage (18) destinée à générer un flux de l'échantillon liquide (16) allant et venant le long du canal de réception (12), communiquant le changement d'orientation de la particule de mesure (14) et un dispositif de détection (20, 22) pour une détection de préférence optique du changement d'orientation de la particule de mesure (14), **caractérisé en ce qu'**une unité d'analyse (56) disposée pour déterminer un paramètre de test conformément au changement d'orientation de la particule de mesure (14) détecté en fonction du temps.

2. Appareil selon la revendication 1, **caractérisé en ce que** l'unité de pompage (18) comporte un organe de déplacement (44) actionné plus particulièrement de façon mécanique, pneumatique ou piézoélectrique pour une aspiration ou une pressurisation changeant de façon cyclique de l'échantillon liquide (16) dans le canal de réception (12).

3. Appareil selon la revendication 1 ou 2, **caractérisé en ce que** l'unité de pompage (18) comporte une membrane de déplacement (28) flexible limitant partiellement le canal de réception (12).

4. Appareil selon l'une quelconque des revendications 1 à 3, **caractérisé en ce que** l'unité de pompage (18) est reliée au niveau d'un point de pompage dans la zone d'une section d'extrémité (34) fermée ou refermable du canal de réception (12).

5. Appareil selon l'une quelconque des revendications 1 à 4, **caractérisé en ce que** le canal de réception (12) est formé par un évidement dans une couche intermédiaire (26) d'un corps composé en feuilles (10) multicouche.

6. Appareil selon l'une quelconque des revendications 1 à 5, **caractérisé en ce que** le canal de réception (12) est formé au moins sous forme de section comme canal capillaire (30).

7. Appareil selon l'une quelconque des revendications 1 à 6, **caractérisé en ce que** les particules de mesure (14) comportent une forme aplatie et/ou allongée éloignée de la forme sphérique.

8. Appareil selon l'une quelconque des revendications précédentes 1 à 7, **caractérisé en ce que** le rapport des mesures longitudinales à transversales des particules de mesure (14) est supérieur à 2, de préférence supérieur à 10.

9. Appareil selon l'une quelconque des revendications 1 à 8, **caractérisé en ce que** les particules de mesure (14) sont formées comme pigments en forme de lamelles, de préférence comme pigments à effets.

10. Appareil selon l'une quelconque des revendications 1 à 8, **caractérisé en ce que** les particules de mesure (14) ont la forme de baguettes ou de fibres, plus particulièrement de barbes.

11. Appareil selon l'une quelconque des revendications 1 à 10, **caractérisé en ce que** les particules de mesure (14) sont enveloppées d'une couche de protection polymère ou oligomère.

12. Appareil selon l'une quelconque des revendications 1 à 11, **caractérisé en ce que** les particules de mesure (14) sont en suspension comme substance étrangère dans l'échantillon liquide (16).

13. Appareil selon l'une quelconque des revendications 1 à 11, **caractérisé en ce que** les particules de mesure (14) sont formées par un composant d'échantillon de l'échantillon liquide (16), plus particulièrement par des érythroses dans un échantillon de sang complet.

14. Appareil selon l'une quelconque des revendications 1 à 13, **caractérisé en ce que** le dispositif de détection (20, 22) comprend une source de rayonnement (20) et un récepteur de rayonnement (22) pour un rayonnement électromagnétique.

15. Appareil selon l'une quelconque des revendications 1 à 14, **caractérisé en ce que** le dispositif de détection (20, 22) comprend une diode lumineuse (20) et un capteur semi-conducteur (22) optoélectronique.

16. Appareil selon l'une quelconque des revendications 1 à 15, **caractérisé en ce que** les particules de mesure (14) absorbent et/ou reflètent différemment le rayonnement électromagnétique émis en fonction de l'angle de rayonnement.

17. Appareil selon l'une quelconque des revendications 1 à 16, **caractérisé en ce que** les parois du canal de réception (12) sont transparentes au moins dans une zone pour le rayonnement de mesure du dispositif de détection (20, 22).

18. Appareil selon l'une quelconque des revendications 1 à 17, **caractérisé en ce que** le canal de réception (12) est couplé à un dispositif d'équilibrage de la température (48) pour définir une température prédéterminée de l'échantillon liquide (16).

19. Procédé pour étudier les propriétés d'écoulement d'un échantillon liquide physiologique, plus particulièrement d'un échantillon de sang pour des tests de coagulation, pour lequel un échantillon liquide (16) est introduit dans un canal de réception (12), des particules de mesure (14) réparties dans l'échantillon liquide (16) sont orientées différemment de façon cyclique, et le changement d'orientation des particules de mesure (14) est de préférence détecté de façon optique, **caractérisé en ce qu'**un flux allant et venant dans le canal de réception (12) de l'échantillon liquide (16) est généré pour permettre une orientation variable cyclique des particules de mesure (14).

20. Procédé selon la revendication 19, **caractérisé en ce que** l'échantillon liquide (16) est soumis à un écoulement de cisaillement dans le canal de réception (12), de sorte que les particules de mesure (14) sont orientées différemment selon le sens du flux.

21. Procédé selon la revendication 19 ou 20, **caractérisé en ce que** le changement d'orientation des particules de mesure (14) est détecté comme modification alternante dans la capacité de réflexion ou d'absorption de l'échantillon liquide (16) au moyen d'un dispositif de détection optique (20, 22).

22. Procédé selon l'une quelconque des revendications 19 à 21, **caractérisé en ce que** le changement d'orientation des particules de mesure (14) se **caractérise par** un grand nombre de cycles et est analysé pour déterminer un ratio, plus particulièrement le temps de prothrombine (PT), le temps de coagulation active (ACT) ou le temps de thromboplastine actif partiel (aPTT) en fonction de la durée.
